# EUROPEAN PATENT APPLICATION

(11) **EP 4 568 424 A1**
(43) Date of publication of application: **11.06.2025**
(21) Application number: 23214735.5
(22) Date of filing: 06.12.2023
(51) Int. Cl.: H05B 47/11, A61B 90/30, H05B 47/125

(54) **A METHOD FOR DETERMINING VISIBLE LIGHT INTENSITY AT AN ILLUMINATION ZONE OF A SURGICAL LIGHT MODULE**

(71) Applicant: Baxter Medical Systems GmbH + Co. KG, 07318 Saalfeld (DE)
(72) Inventor: COSKUN, Tayfur, 07318 Saalfeld (DE); ALTMANN, Maximilan, 07318 Saalfeld (DE)
(74) Representative: Reddie & Grose LLP

(57) **Abstract**

There is provided a method for determining visible light intensity at an illumination zone of a surgical light module. The surgical light module comprising a visible light system and a structured light system. The method comprises detecting an interference pattern, generated by visible light emitted by the visible light system and infrared light emitted by the structured light system, at the illumination zone. The method further comprises determining a visible light intensity value at the illumination zone, comprising inputting the detected interference pattern into an interference pattern recognition algorithm. The interference pattern recognition algorithm is generated using known interference patterns and corresponding light intensities.

## Description

### Field of the Disclosure

The present disclosure relates to a method for determining visible light intensity at an illumination zone of a surgical light module, and a surgical light module. In particular, the present disclosure relates to a method of determining visible light intensity at an illumination zone of a surgical light module comprising a visible light system, a structured light system and an interference pattern recognition algorithm.

### Background

Surgical lights are lighting fixtures designed for use during surgical procedures, to provide illumination to a desired area. Due to the particular demands in a medical or surgical setting, surgical lights are subject to a large number of requirements in terms of their construction and lighting performance. IEC60601-2-41 (IEC60601-1-2005) states that total irradiance should be minimized, and that the total irradiance in the lighted area should not exceed a specified amount.

IEC60601-2-41 (IEC60601-1-2005) defines the allowed minimum and maximum light intensity for one surgical light. In order to meet this standard, surgical lights are calibrated during manufacture and production such that they meet the specified light intensity required during operation. In order to determine the light intensity of the surgical light during operation, known surgical light modules comprise additional hardware to be able to determine the real light intensity during the operation of the surgical light. However, this additional hardware is expensive and complex.

It has been appreciated that it would be desirable to provide a more efficient and cost effective method for determining the visible light intensity at an illumination zone of a surgical light module.

### Summary of Disclosure

The present disclosure provides a method of determining visible light intensity at an illumination zone of a surgical light module and a surgical light module as defined in the appended independent claims, to which reference should now be made. Optional and preferred features are set out in the dependent claims.

According to a first aspect of the present disclosure, there is provided a method for determining visible light intensity at an illumination zone of a surgical light module. The surgical light module comprises a visible light system and a structured light system. The method comprises detecting an interference pattern, generated by visible light emitted by the visible light system and infrared light emitted by the structured light system, at the illumination zone. The method further comprises determining a visible light intensity value at the illumination zone, which comprises inputting the detected interference pattern into an interference pattern recognition algorithm, the interference pattern recognition algorithm being generated using known interference patterns and corresponding light intensities.

The present disclosure advantageously provides a method for determining the visible light intensity value at the illumination zone. This determined intensity may then be used to confirm that the standards with regards to light intensity are complied with, for example. In use, the illumination zone will most likely be at the surface of a surgical table and/or a patient support surface.

Further, the method according to the first aspect only requires a single surgical light module to determine the visible light intensity at the illumination zone, whereas known prior art devices require overlapping illumination zones of at least two surgical light modules in order to accurately determine the visible light intensity. However, the method according to the present disclosure can also be applied in the event more than one surgical light module is present.

The interference pattern recognition algorithm is configured to receive the detected interference pattern, generated by visible light emitted by the visible light system and infrared light emitted by the structured light system, at the illumination zone, and determine, and in turn output, a visible light intensity value. The interference pattern recognition algorithm is generated and trained using known interference patterns and corresponding light intensity values. In other words, the algorithm is trained to recognise that particular interference patterns are associated to specific visible light intensity values. This training of the algorithm may be carried out during calibration at a factory or manufacturing site. Alternatively, the training of the algorithm may occur both during the calibration stage but also continually throughout operation of the surgical light module. The interference pattern recognition algorithm advantageously provides an efficient and accurate method for determining a visible light intensity value. The method advantageously allows continuous checks, even after years of operation, to confirm that the real-time light intensity value meets the initial intensity value set in the factory or when first implemented into the surgical environment. This further advantageously means that if the system has an error or bug, for example, that causes the intensity to reduce or increase significantly, the surgical light module is continuously able to re-evaluate its own light intensity in comparison to the expected light intensity value.

The method according to the first aspect may advantageously provide a method for determining visible light intensity at an illumination zone of a surgical light module that does not require additional and complex hardware. Instead, the method can utilise existing surgical light modules such as a surgical light module which is marketed by Baxter Medical Systems GmbH + Co. KG as iLED° 7 and Helux Pro. Providing a method which can be implemented on certain existing surgical light modules in turn also advantageously reduces manufacturing costs and time.

Optionally, the interference pattern recognition algorithm is an artificial neural network. Advantageously, using an artificial neural network allows for improved determination of the visible light intensity value.

Additionally, when provided, the artificial neural network may be configured to receive and process context-sensitive information based on at least one of: a patient characteristic; a surgery type; environmental data; and illumination settings, and/or focus settings, of the surgical light module. This advantageously allows the artificial neural network to be dynamic and adjust based on this context-sensitive information and in particular, may provide a more context specific output based on a particular scenario.

Optionally, the artificial neural network is generated using a training set of data which comprises a plurality of images of interference patterns each at a known light intensity. This advantageously provides an artificial neural network that can more accurately determine the visible light intensity based on the detected interference pattern. The training set of data may be dependent on the particular surgical light module and in particular the specific light intensity standards applicable to the particular surgical light module. Further, the training set of data may be generated by detecting the interference pattern in different scenarios where the light intensity might vary. For example, detecting the interference pattern at each light intensity, where a distance between the structured light system and the illumination zone is varied and/or for different patient types (i.e., different ages and/or sizes), and/or with differing surrounding lighting and/or for a range of different sized illumination zones.

Optionally, the method further comprises comparing the determined light intensity value to an expected light intensity at a light intensity set point of the surgical light module. Upon a difference between the determined light intensity value and the expected light intensity at the light intensity set point of the surgical light module exceeding a first threshold, the method may further comprise adjusting the visible light system such that the determined light intensity matches the expected light intensity.

The light intensity set point of the surgical light module being the setting of the surgical light module. For example, the user may set the surgical light module to light intensity set point 2 which corresponds to an expected light intensity value of about 40 klx to 60 klx. Alternatively, the user may set the surgical light module to light intensity set point 3 which corresponds to an expected light intensity value of about 70 klx to 90 klx. This expected light intensity value is the light intensity value that the neural network expects to determine for that light intensity set point.

This advantageously provides a surgical light module, and a visible light system, as well as a method for operating the module and system, that can correct or adjust in operation / real-time to ensure the required visible light intensity value is achieved. The method is able to compare the determined light intensity value to an expected light intensity at a light intensity set point of the surgical light module to assess whether the difference between the two has exceeded a predetermined first threshold. This advantageously provides a surgical light module with a mechanism to ensure that the required visible light intensity for that particular light intensity set point is being met at the illumination zone.

Adjusting the visible light system such that the determined light intensity matches the expected light intensity for that light intensity set point may be automatic and/or may occur in steps or increments.

Adjusting the visible light system may comprise adjusting an electric power provided to the visible light system, or a portion of the light system, or adjusting a focus of the visible light system.

Optionally, the first threshold is between about 5 % and about 25 % of the light intensity at the set point. Preferably, the first threshold is between about 10 % and 20 % of the light intensity at the set point. More preferably, the first threshold is about 15 % of the light intensity at the set point. Alternatively, the first threshold is between about 5 % and about 25 %, or about 10 % and 20 %, or about 15 %, of the expected light intensity.

Optionally, the method further comprises outputting an indication to an indication system upon a difference between the determined light intensity value and the expected light intensity at the light intensity set point of the surgical light module exceeding the first threshold. This may advantageously indicate to a user or operator that the determined light intensity value is not meeting the expected light intensity value at the light intensity set point of the surgical light module. This indication may not necessarily require the user or operator of the surgical light module to carry out any action, instead, the indication may be outputted to indicate that this difference has been detected and that the system is re-adjusting the surgical light module so that the determined light intensity meets the expected light intensity value at the light intensity set point. However, in some embodiments the indication may require some action from the user to show they have acknowledged the indication.

Optionally, the method further comprises comparing the determined light intensity value to an expected light intensity at a light intensity set point of the surgical light module. Upon a difference between the determined light intensity value and the expected light intensity at the light intensity set point of the surgical light module exceeding a second threshold, the method further comprises outputting a maintenance indication. Outputting a maintenance indication occurs when the difference between the determined light intensity value and the expected light intensity at the light intensity set point of the surgical light module exceeds the difference whereby the visible light system can be adjusted such that the determined light intensity value equals the expected light intensity value at a light intensity set point of the surgical light module. Therefore, in this case, it is necessary to output a maintenance indication to indicate that the surgical light module needs maintenance or needs to be replaced.

The method may further require an acknowledgement of the maintenance indication. In particular, the method may require an acknowledgement of the maintenance indication before the light set point, or an illumination setting of the surgical light module, may be changed.

Optionally, the second threshold is greater than the first threshold. This means that in use, and where both the first threshold and the second threshold are present, before the system outputs a maintenance indication it will have attempted to re-calibrate, correct or adjust the visible light system such that the determined light intensity value is the same as the expected light intensity value at the light intensity set point of the surgical light module. Advantageously, this may reduce the number of maintenance indications outputted and in turn may reduce the required maintenance resources such as engineers at the site. This may further advantageously reduces the maintenance costs.

The second threshold may be greater than about 70 %, or greater than about 50 %, or greater than about 40%, of the expected light intensity at the set point.

Optionally, where outputting an indication comprises outputting at least one of: a visual indication; and an audible indication. This may allow for an indication to be output clearly, and for it to be quickly received, by a user.

Optionally, where outputting an indication comprises outputting an indication on at least one of: the surgical light module; a wall control panel; a mobile controller; and a third party device.

For example, the surgical light module may comprise indicia to display the indication. For example, an indication may be displayed by illuminating the indicia. The indicia may be illuminated in specific colour. The colour may be a colour that makes the indication noticeable, e.g. red, yellow or orange.

The wall control panel may comprise a Graphical User Interface (GUI). The indication may be output within the GUI. Alternatively or additionally, the mobile controller may comprise a, or the, GUI.

Additionally, the indication may comprise context-sensitive information such as at least one of: a patient characteristic; surgery type; environmental data; and illumination settings, and/or focus settings, of the surgical light module. Advantageously, including such context-sensitive information may provide for improved and/or guided human-machine interaction and may allow the user to easily see relevant context-sensitive information.

Optionally, the method further comprises comparing the determined light intensity value to an expected light intensity at a light intensity set point of the surgical light module. Upon the determined light intensity value being greater than the expected light intensity at the light intensity set point of the surgical light module, the method may further comprise calibrating the structured light system to adjust a determined distance between the surgical light system and the illumination zone. This advantageously allows the system to calibrate the structured light system in the event the determined light intensity value is incorrect, i.e., it is greater than the expected light intensity at the light intensity set point of the surgical light module. In particular, it has been appreciated that this error in determining light intensity value often occurs when the determined distance between the surgical light system and the illumination zone is incorrect, i.e., the distance determined by the structured light system. This is because the determined distance between the surgical light system and the illumination zone is an input for the determined light intensity value. Advantageously, providing a method with this mechanism for calibrating the determined distance in use further reduces the frequency of false maintenance indications being outputted and in turn reduces the resources and costs with regards to maintenance.

Optionally, the step of detecting the interference pattern comprises capturing an image of the interference pattern. This captured image of the interference pattern may then be inputted into, or provided as an input to, the interference pattern recognition algorithm.

Optionally, the method further comprises using a structured light sensor of the structured light system to capture the image of the interference pattern. Using the structured light sensor of the structured light system advantageously uses pre-existing hardware of the structured light system to capture the image of the interference pattern.

According to a second aspect of the present disclosure, there is provided a surgical light module comprising a visible light system, a structured light system, and a computer device for determining visible light intensity at an illumination zone of the surgical light module. The computer device comprising a processing device and a memory storing instructions that, when executed by the processing device, cause the computer device to: detect an interference pattern, generated by visible light emitted by the visible light system and infrared light emitted by the structured light system, at the illumination zone; and determine a visible light intensity value at the illumination zone, comprising inputting the detected interference pattern into an interference pattern recognition algorithm, the interference pattern recognition algorithm being generated using known interference patterns and corresponding light intensities.

The memory can store instructions which, when executed by the processor, enable the computer device to carry out the steps it is configured to execute. The computer device may be connected to the surgical light module via a wired or wireless connection. Alternatively, the computer device may be integral to the surgical light module.

The computer device, in particular the interference pattern recognition algorithm, may be configured to continually determine the visible light intensity value. This advantageously allows for up-to-date indications to be provided. Alternatively, the computer device may be configured to determine the visible light intensity value at predetermined time intervals. The predetermined time interval may be between 1 second and 1 minute, or between 1 second and 10 seconds, or between 1 second and 5 seconds. Increasing the time interval may reduce the computational burden, and therefore increase the efficiency of the system.

Optionally, the instructions stored in the memory, which when executed by the processing device, further cause the computer device to implement an artificial neural network configured to be, or process, the interference pattern recognition algorithm. Advantageously, using an artificial neural network allows for improved determination of the visible light intensity value.

Optionally, the surgical light module comprises a controller configured to receive an input for increasing and/or decreasing the visible light emitted by the visible light system. The controller may further be configured to communicate said input to the computer device.

Optionally, the surgical light module may comprise at least one of: a position sensor; an angle sensor; an accelerometer; and a gyroscope, for determining the position and orientation of the, or each, module relative to a patient support surface.

Optionally, the surgical light module comprises an indication system. The indication system may comprises an indicator. An indication may be output by activating the indicator. The indicator may be a visual indicator and/or an audible indicator. The indication may be output on at least one of: the surgical light module; a wall control panel; a mobile controller; and a third party device. This may allow for an indication to be output clearly, and for it to be quickly received by a user.

Optionally, the surgical light module comprises a light module control panel which comprises indicia. The wall control panel may comprise a Graphical User Interface (GUI). The indication may be output within the GUI. Alternatively or additionally, the mobile controller may comprise a, or the, GUI.

Optionally, the computer device may be configured to receive at least one of: inputs for acknowledging the indication; inputs for putting the indication system on hold; and inputs for pausing the indication. The inputs for putting the indication on hold may comprise setting a period of time the indication system is put on hold, and/or setting an event to trigger reactivation of the indication system. As will be appreciated, the indication may be "paused" only after it has been activated, whereas the indication system may be put on hold at any time. Such inputs allow for a more efficient indication system in which a user may interact with the indication in a number of improved ways.

According to a third aspect of the present disclosure, there is provided a surgical light apparatus comprising: a surgical light module according to the second aspect; and at least one further surgical light module. Optionally, the or each at least one further module may be according to the second aspect, or may differ from a surgical light module according to the second aspect in one or more respects.

Where functional components are referred to in apparatus embodiments for carrying out various steps of the described method(s) it will be understood that these components may be implemented in hardware, in software, or a combination of the two. When implemented in hardware, the components may be implemented as one or more hardware components, such as one or more application specific integrated circuits. When implemented in software, the components may be implemented as one or more computer programs that are executed on one or more processors.

It will be appreciated that features described in relation to one aspect of the present disclosure may also be applied equally to all of the other aspects of the present disclosure. Features described in relation to the first aspect of the present disclosure may be applied equally to the second and third aspects of the present disclosure and vice versa. For example, the method steps described in relation to the first aspect may be applied, mutatis mutandis, to the surgical light module of the second aspect. In particular, the surgical light module of the second aspect may be configured to, or the memory may store instructions, to carry out any of the method steps of the first aspect.

### Brief Description of Drawings

The disclosure will be further described, by way of example only, with reference to the accompanying drawings, in which:
Figure 1 shows a schematic of a surgical lighting apparatus comprising a surgical light module according to the present disclosure;
Figure 2 shows a flow diagram of a method according to the present disclosure;
Figure 3 shows a flow diagram of a further example method according to the present disclosure;
Figures 4a to 4e shows a plurality of images of interference patterns which may be used as training data;
Figure 5 shows a schematic illustration of an artificial neural network;
Figure 6 shows a flow diagram of a further example method according to the present disclosure;
Figures 7a, 7b, and 7c show example light module control panels for a surgical light module;
Figures 8a and 8b show example Graphical User Interfaces (GUIs); and
Figure 9 shows a flow diagram of a further example method according to the present disclosure.

### Specific Description

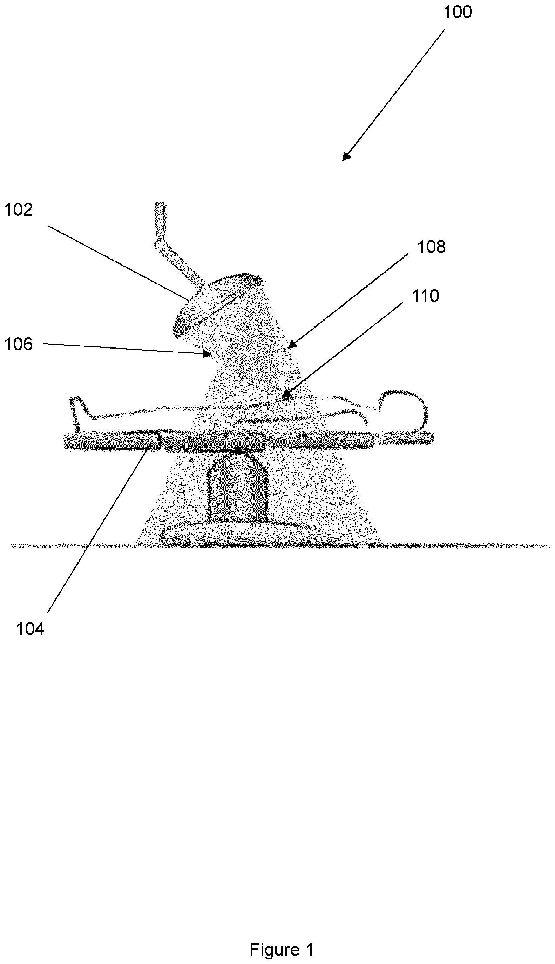

Figure 1 shows a schematic illustration of a surgical lighting apparatus 100 comprising a surgical light module 102. However, it will be appreciated that the surgical light apparatus 100 may comprise two, three, or more surgical light modules, which may be identical to, or different from, the surgical light module 102. The, or each, surgical light module 102 is configured to illuminate a surgery site on a patient disposed on a patient support apparatus 104.

The surgical light module 102 comprises a visible light system 106 which emits visible light. The surgical light module 102 further comprises a structured light system 108 which emits infrared light. The visible light emitted by the visible light system 106 and the infrared light emitted by the structured light system 108 produce an interference pattern at the illumination zone 110.

The structured light system 108 provided in the surgical light module 102 comprises a structured light sensor. The structured light sensor is able to capture the image of the interference pattern at the illumination zone 110. The surgical light module 102 may be a light module marketed by Baxter Medical Systems GmbH + Co. KG as iLED° 7 or Helux Pro. The structured light sensor of the example embodiment is a camera. The camera may be a standard camera, such as RGB cameras using a CCD or CMOS type sensor.

Although not shown, the surgical light module 102 comprises a computer device. The computer device may be connected to the surgical light module 102 via a wired or a wireless connection. Alternatively, the computer device may be integral the surgical light module 102, to a wall control panel connectable to the computer device, or to a hospital computer system such as an Electronic Health Record system, or to a third party device such as a tablet.

The computer device comprises a processing device and a memory storing instructions that, when executed by the processing device, cause the computer device to detect an interference pattern, generated by visible light emitted by the visible light system 106 and infrared light emitted by the structured light system 108, at the illumination zone 110.

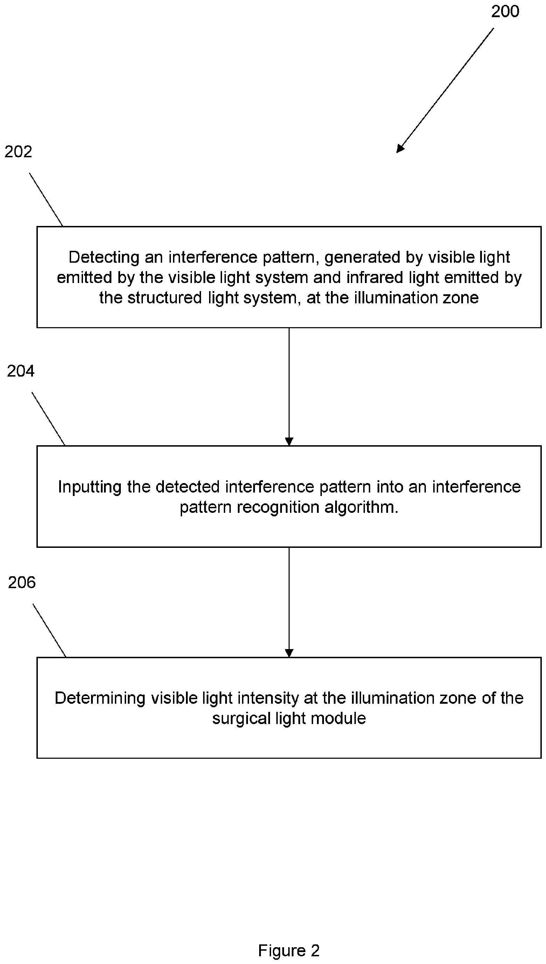

Figure 2 is a flow diagram of a method 200 for determining visible light intensity at the illumination zone 110 of the surgical light module 102 according to the present disclosure. The method 200 comprising detecting 202 an interference pattern, generated by visible light emitted by the visible light system 106 and infrared light emitted by the structured light system 108, at the illumination zone 110. As discussed above, in the example embodiment, this step of detecting 202 an interference pattern is carried out by the structured light sensor of the structured light system 108. The structure light sensor of the structure light system 108 captures an image of the interference pattern. The method 200 further comprises the step of determining 206 a visible light intensity value at the illumination zone. The visible light intensity value at the illumination zone is determined by inputting 204 the detected interference patten into an interference pattern recognition algorithm. In this example embodiment, the interference pattern recognition algorithm is an artificial neural network.

The artificial neural network is generated using a training set of data. The training set of data comprises a plurality of images of interference patterns each at a known light intensity.

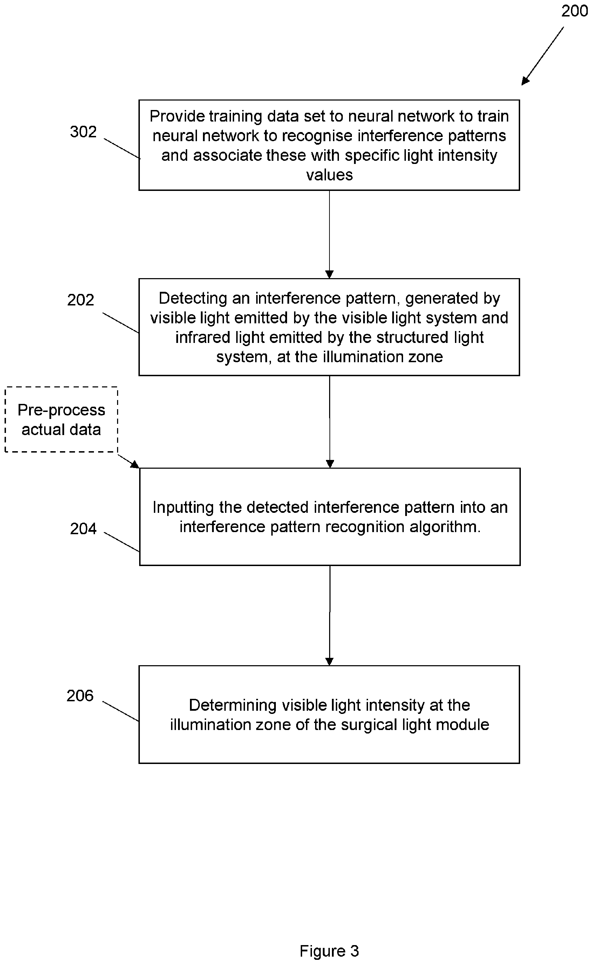

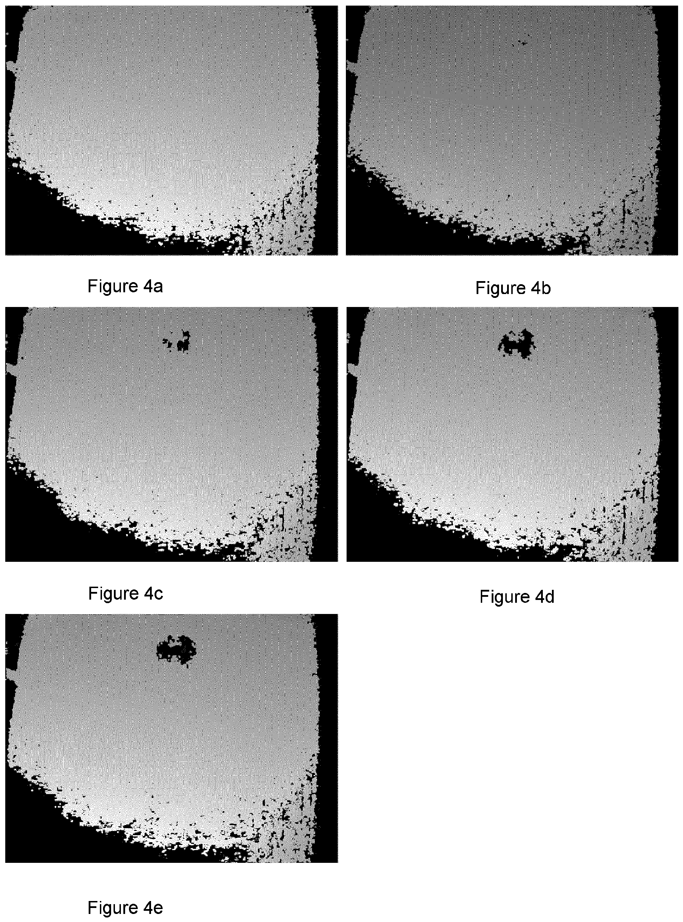

As set out in Figure 3, the method 200 further comprises the step of providing 302 training data set to train the neural network to recognise interference patterns from the visible light emitted from the visible light system and infrared light emitted from the structured light system and associate these interference patterns with particular visible light intensity values. For example, Figures 4a to 4e show interference patterns for different visible light intensity values.

**Table 1: Interference patterns for different visible light intensity values**

| Figure | Percentage | Light Intensity (klx) |
|---|---|---|
| 4a | 10% | 16 |
| 4b | 30% | 48 |
| 4c | 50% | 80 |
| 4d | 80% | 128 |
| 4e | 100% | 160 |

The above-mentioned light intensity values are examples only and as such it will be appreciated that alternative values may also be provided. The light intensity values have a tolerance of +/- 10%, except 100% shown in Figure 4e which is the maximum allowed light intensity with no tolerance.

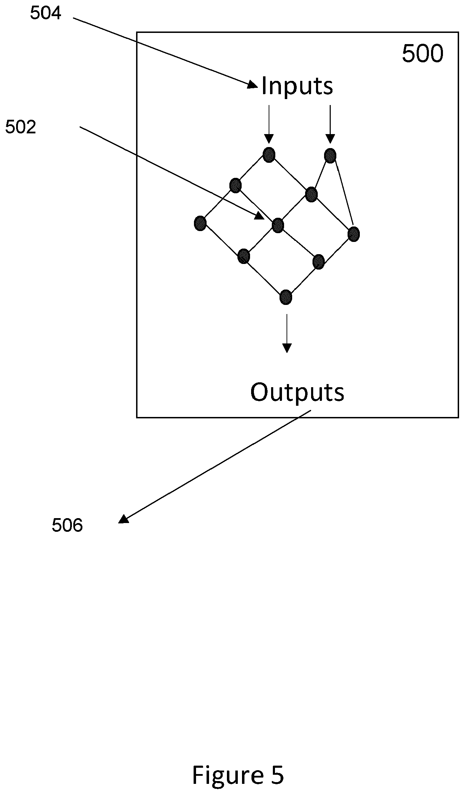

As shown in Figures 5, the artificial neural network 500 may be trained using a training data set 502. The training data set 502 may comprise labelled data. The labelled data includes pairs consisting of an input and a desired output. Upon training using the training data set 502, each node layer in the neural network 500 is configured to automatically learn to recognise features of the training data 502 set by repeatedly trying to reconstruct the input (i.e. the training data set). The neural network 500 learns by trying to minimise the difference between the network's output and the probability distribution of the training data set 502.

The labelled data of the training data set 502 comprises inputs 504 such as images from cameras, e.g. structured light images from the structured light systems, or RGB images from standard cameras. While this example relates to supervised machine learning to generate the neural network, the skilled person would understand that, where appropriate, semi-supervised machine learning, or other machine learning may be used.

The training data set 502 may be generated in conditions which are close to real conditions i.e. conditions during use. The training data set 502 may be generated in different scenarios and / or conditions. For example, the plurality of images of the interference pattern at a certain light intensity value can be obtained when the distance between the surgical light module and the patient support surface is varied. Further, images of the interference pattern may be obtain for a certain light intensity value when the patient type is varied, i.e., when the patient type is an adult compared to a child. Further still, the training data set 502 may include images of the interference patterns each at a known light intensity when there is more than one surgical light module. Even further still, the training data set 502 may include images of the interference patterns each at a known light intensity with differing ambient lighting based on the operating room for example. Other suitable conditions for the training data set 502 will be appreciated by the skilled person.

Once the neural network 500 has been trained, actual data, such as data from the structured light system and in particular cameras, is used as the input 504. The data being the detected interference pattern at the illumination zone captured by the structured light sensor. The output 506 is the visible light intensity value associated with the detected interference pattern based on the training data set 502. The neural network 500 is configured to recognise corresponding features, that is, features present in the images of certain visible light intensity values. In other words, the algorithm is configured to map real-time interference patterns to the corresponding image in training data set. This will in turn provide the visible light intensity value.

The neural network 500 may be developed using an open source machine learning platform such as TensorFlow, or PyTorch.

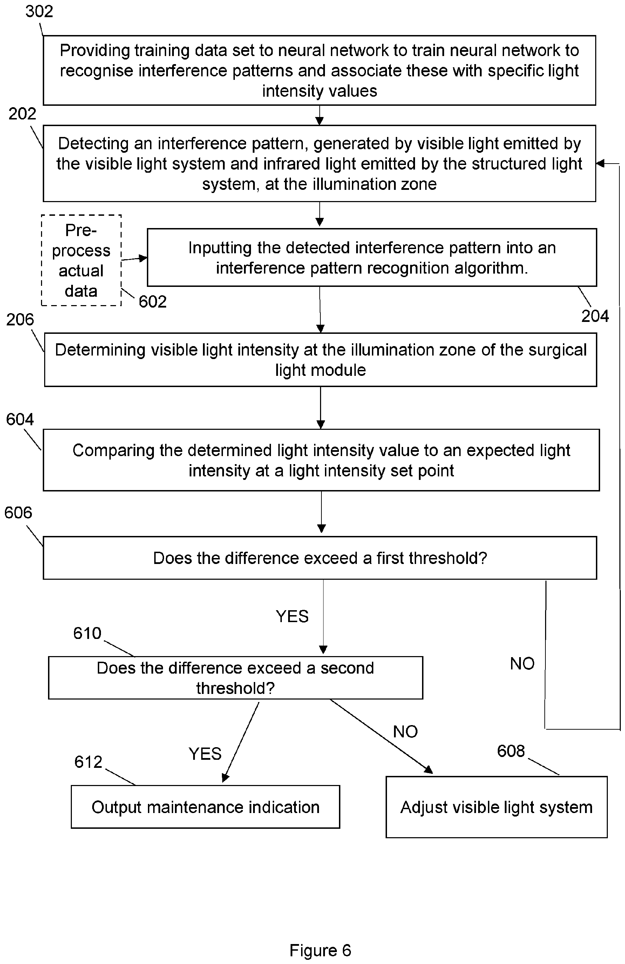

As shown in Figure 6, the method 600 may further comprise pre-processing actual data 602. Therefore, the actual input data may be pre-processed to facilitate processing of the actual data by the neural network 500. For example, the actual data may be pre-processed by at least one of: cropping the input images of the interference pattern; and reducing a resolution of the input images to increase the performance of overlap detection. Cropping the input may include cropping the input to correspond to the portion of the input corresponding to the corresponding illumination zone.

The neural network 500 may be configured to continue training after the initial training with the training data set 502. For example, intermittently, the neural network 500 may request confirmation that the result of the visible light intensity value is correct. Additionally or alternatively, the neural network 500 may be intermittently provided with additional training data, comprising inputs and desired outputs, to improve the neural network 500.

As shown in Figure 6, the method 600 further comprises a step of comparing 604 the determined light intensity value to an expected light intensity at a light intensity set point of the surgical light module 102. The light intensity set point of the surgical light module 102 being the setting of the surgical light module 102, for example, the user may set the surgical light module 102 to light intensity set point 2 which corresponds to an expected light intensity value of 48 klx. Alternatively, the user may set the surgical light module 102 to light intensity set point 3 which corresponds to an expected light intensity value of 80 klx. This expected light intensity value is the light intensity value that the neural network expects to determine for that light intensity set point.

The method 600 further comprises assessing or determining 606 whether the difference between the determined light intensity value and the expected light intensity at the light intensity set point of the surgical light module exceeds a first threshold. Upon the difference between the determined light intensity value and the expected light intensity at the light intensity set point of the surgical light module exceeding a first threshold, the method further comprises adjusting 608 the visible light system such that the determined light intensity matches the expected light intensity for that light intensity set point. The first threshold is between about 5 % and about 25 % of the expected light intensity at the set point. Therefore, if the difference between the light intensity value determined in real time and the expected light intensity for that light intensity set point exceeds between about 5% and 25% of the expected light intensity value at the light intensity set point the surgical light module is configured to adjust the visible light system such that the light intensity of the surgical light module in operation substantially matches or equals the expected light intensity. Adjusting the visible light system such that the determined light intensity matches the expected light intensity for that light intensity set point may be automatic and/or may occur in steps or increments, as the artificial neural network will be continuously output the determined light intensity value. If the difference does not exceed the first threshold, then the method returns to the step of detecting 202 an interference pattern.

The method may further comprise the step of outputting an indication to an indication system upon a difference between the determined light intensity value and the expected light intensity at the light intensity set point of the surgical light module exceeding the first threshold. The indication may be at least one of a visual indication and an audible indication, as discussed in more detail below.

The method may further comprise the step of comparing 604 the determined light intensity value to an expected light intensity at a light intensity set point of the surgical light module, as discussed. However, the method further comprises comparing 610 this difference to a second threshold. Upon the difference between the determined light intensity value and the expected light intensity at the light intensity set point of the surgical light module exceeding a second threshold, the method may further comprise outputting 612 a maintenance indication. The second threshold is greater than the first light intensity threshold. The second threshold may be greater than 25% of the expected light intensity at the set point. The second threshold is a value at which maintenance of the surgical light is required and adjusting the determined light intensity value is not suitable. The maintenance indication may be at least one of a visual indication and an audible indication, as discussed in more detail below. If the difference does not exceed the second threshold but exceeds the first threshold then the step of adjusting 608 the visible light system such that the determined light intensity matches the expected light intensity for that light intensity set point is carried out.

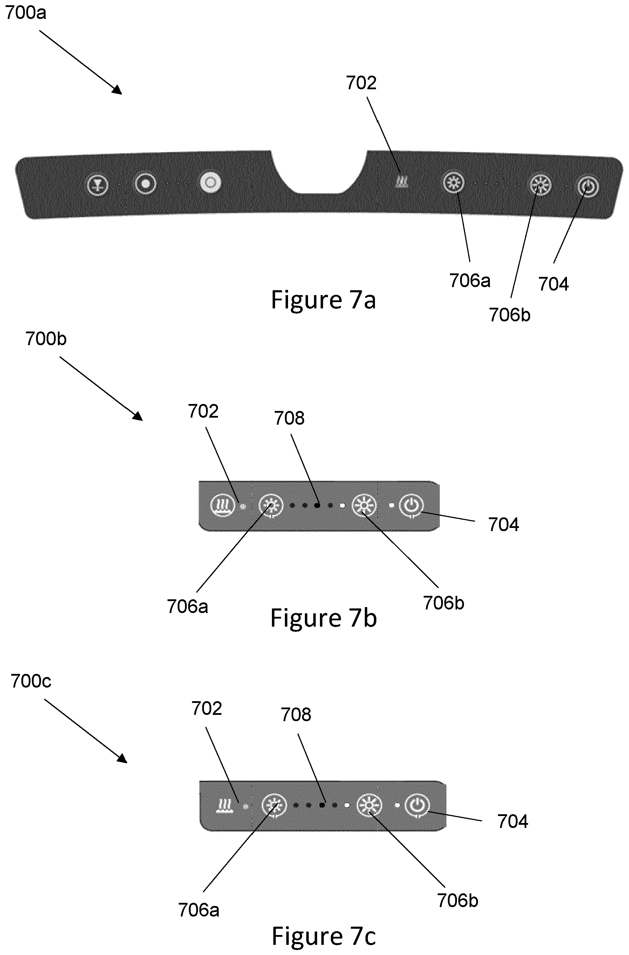

Figure 7a shows an example of a light module control panel 700a of the surgical light module 102 having an icon which may be illuminated to output a visual indication. In some embodiments, the icon 702 may be a button which may be pressed to acknowledge the indication, in particular, when the maintenance indication is generated this may require an operator or an engineer to acknowledge the indication. However, generally, if the indication is outputted in response to the first threshold being exceeded and as a result the light intensity value is adjusted to match the expected light intensity value an acknowledgment to the indication should not be required. The surgical light module may be configured to remove the indication once the necessary action has been taken.

The light module control panel further comprises an input, in the form of a button 704, to switch the surgical light module 102 between on and off, and inputs, in the form of buttons 706a, 706b for selecting the light intensity set point or increasing and decreasing an illumination setting of the surgical light module 102.

Figures 7b and 7c show further examples of the light module control panel 700b, 700c, wherein instead of the indication icon 702 being illuminated, a light adjacent the icon may be illuminated to output the indication. The light module control panels 700b, 700c may further comprise an illumination setting indicator 708, which comprises a number of indicia to indicate a current illumination setting of the surgical light module 102.

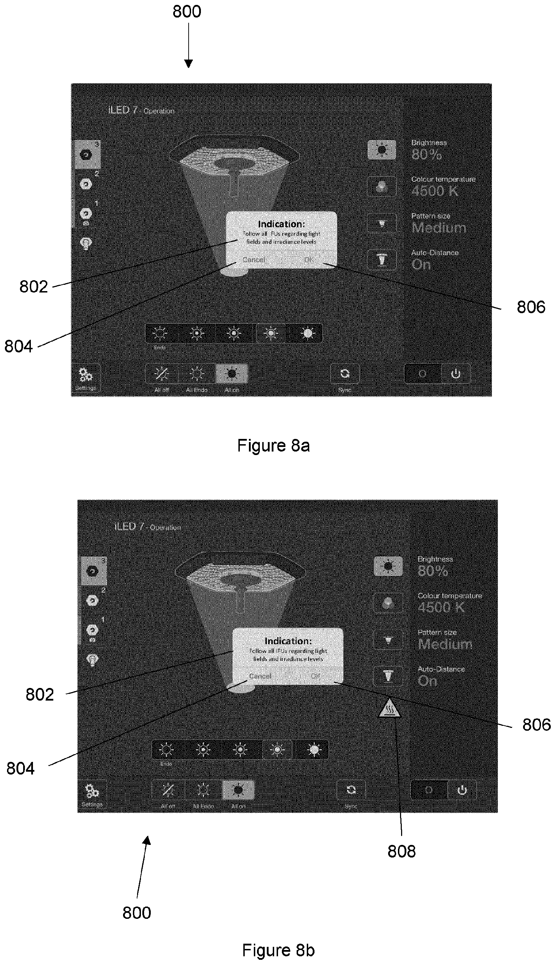

Figures 8a and 8b show examples of Graphical User Interfaces (GUI) 800 for outputting the indication. The GUI 800 may be displayed on the wall control panel and/or on a third party device such as a tablet.

The indication may be displayed within the GUI 800 as a pop-up window 802. The pop-up window 802 may include one or more buttons for acknowledging the indication. In the embodiments of Figures 8a and 8b, the GUI comprises a "Cancel" button 804 which may cause an automatic reduction in the illumination setting of the surgical light module 102, or may simply cause removal of the indication, and an "OK" button 806 which only acknowledges the indication, and may cause the requested increase in the illumination setting to be initiated.

In addition, the pop-up window 802 may provide a link, such as a hyperlink, to the relevant instructions for use (IFUs), and in particular the link may be to the particular page or section in the relevant instructions for use. On initiation of the link a further pop-up window (not shown) may be provided with the relevant instructions for use for immediate review by the user. Alternatively, on initiation of the link, the system may provide access to the relevant instructions for use using the standard user interface for the instructions for use.

In addition to the pop-up window 802, Figure 8b of the example GUI 800 shows an example of an additional indicator 808, which may be displayed to draw further attention to the indication. Even after acknowledgement of the indication pop-up window 802, the additional indicator 808 may still be displayed in the GUI 800.

The indication may be displayed on more than one of the surgical light module control panel, a mobile controller, a third party device, and the wall control panel. In particular, there may be provided a LED light system on the surgical light module which illuminates with colours to indicate certain scenarios.

The indication may additionally or alternatively be an audible indication such as an alarm sound. There may be an audio or speech output from the surgical light module or the wall control panel and/or on a third party device such as a tablet.

The indication may additionally or alternatively be an automatic communication, for example, an email to maintenance personal who can then inform the user or carry out the necessary maintenance on the surgical light module.

The indication may further additionally be a visual feedback through LEDs on the surgical light module.

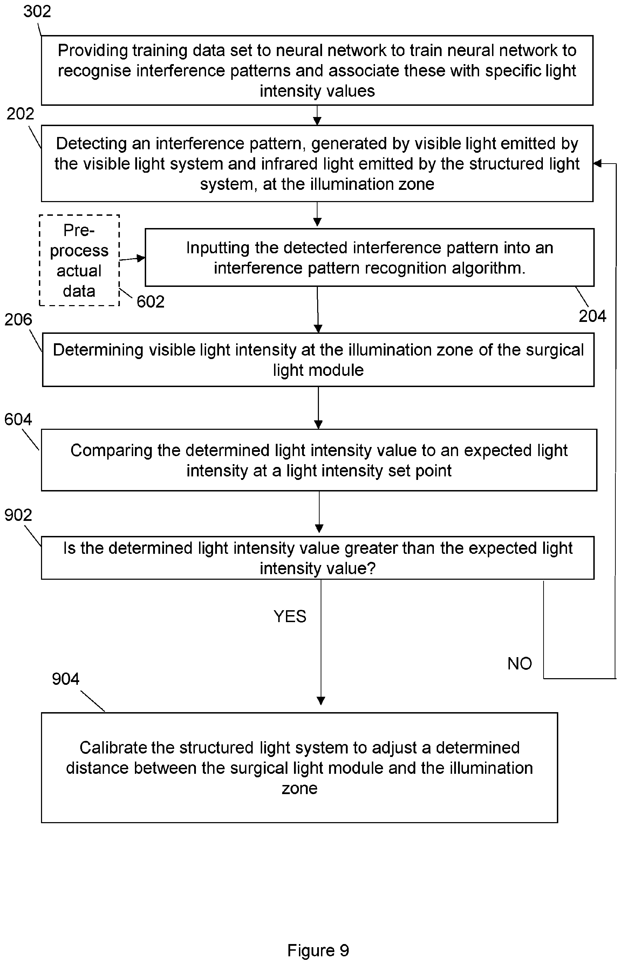

As shown in Figure 9, the method may further comprise the step of comparing 902 the determined light intensity value to an expected light intensity at a light intensity set point of the surgical light module. Where upon the determined light intensity value being greater than the expected light intensity at the light intensity set point of the surgical light module, the method further comprises calibrating 904 the structured light system to adjust a determined distance between the surgical light module and the illumination zone. As discussed above, the structured light system comprises a structured light sensor. This structured light sensor is also able to determine the distance between the surgical light system and the illumination zone. In use, the determined light intensity value should not be greater than the expected light intensity at the light intensity set point, because the expected light intensity at the light intensity set point is factory set accordingly.

Therefore, when the computer device compares the determined light intensity value to the expected light intensity at the light intensity set point of the surgical light module and determines that the determined light intensity value is greater than the expected light intensity at the light intensity set point of the surgical light module, then it has been appreciated that recalibrating the structured light system to adjust the determined distance between the surgical light system and the illumination zone can be at least a first suitable step before outputting a maintenance indication. This is because if the determined distance being inputted by the structured light system into the neural network is incorrect, this could result in an incorrect determined light intensity value output. The calibration may be manual or may be automatic.

It will be appreciated that the above described embodiments are exemplary embodiments of the disclosure only. It will also be appreciated that features described above in relation to one embodiment of the disclosure may also be applied to other embodiments of the disclosure.

## Claims

1. A method for determining visible light intensity at an illumination zone of a surgical light module, the surgical light module comprising a visible light system and a structured light system, the method comprising:
detecting an interference pattern, generated by visible light emitted by the visible light system and infrared light emitted by the structured light system, at the illumination zone; and
determining a visible light intensity value at the illumination zone, comprising inputting the detected interference pattern into an interference pattern recognition algorithm, the interference pattern recognition algorithm being generated using known interference patterns and corresponding light intensities.

2. The method according to claim 1, the interference pattern recognition algorithm being an artificial neural network.

3. The method according to claim 2, wherein the artificial neural network is generated using a training set of data which comprises a plurality of images of interference patterns each at a known light intensity.

4. The method according to any preceding claim, further comprising comparing the determined light intensity value to an expected light intensity at a light intensity set point of the surgical light module, wherein upon a difference between the determined light intensity value and the expected light intensity at the light intensity set point of the surgical light module exceeding a first threshold, the method further comprises adjusting the visible light system such that the determined light intensity matches the expected light intensity.

5. The method according to claim 4, wherein the first threshold is between about 5 % and about 25 % of the light intensity at the set point.

6. The method according to claim 4 or 5, further comprising outputting an indication to an indication system upon a difference between the determined light intensity value and the expected light intensity at the light intensity set point of the surgical light module exceeding the first threshold.

7. The method according to any preceding claim, further comprising comparing the determined light intensity value to an expected light intensity at a light intensity set point of the surgical light module, wherein upon a difference between the determined light intensity value and the expected light intensity at the light intensity set point of the surgical light module exceeding a second threshold, the method further comprises outputting a maintenance indication.

8. The method according to claim 7, when dependent on any of claims 4 to 6, wherein the second threshold is greater than the first threshold.

9. The method according to claim 6 to 8, wherein outputting an indication comprises outputting at least one:
a visual indication; and
an audible indication.

10. The method according to any one of claims 6 to 9, wherein outputting an indication comprises outputting an indication on at least one of:
the surgical light module;
a wall control panel;
a mobile controller; and
a third party device.

11. The method according to any preceding claim, further comprising comparing the determined light intensity value to an expected light intensity at a light intensity set point of the surgical light module, wherein upon the determined light intensity value being greater than the expected light intensity at the light intensity set point of the surgical light module, the method further comprises calibrating the structured light system to adjust a determined distance between the surgical light system and the illumination zone.

12. The method according to any preceding claim, wherein the step of detecting the interference pattern comprises capturing an image of the interference pattern.

13. The method according to claim 12, comprising using a structured light sensor of the structured light system to capture the image of the interference pattern.

14. A surgical light module comprising:
a visible light system;
a structured light system; and
a computer device for determining visible light intensity at an illumination zone of the surgical light module, the computer device comprising a processing device and a memory storing instructions that, when executed by the processing device, cause the computer device to:
detect an interference pattern, generated by visible light emitted by the visible light system and infrared light emitted by the structured light system, at the illumination zone; and
determine a visible light intensity value at the illumination zone, comprising inputting the detected interference pattern into an interference pattern recognition algorithm, the interference pattern recognition algorithm being generated using known interference patterns and corresponding light intensities.

15. The surgical light module according to claim 14, wherein the instructions stored in the memory, which when executed by the processing device, further cause the computer device to implement an artificial neural network configured to be the interference pattern recognition algorithm.
